(19) Europäisches Patentamt
European Patent Office
Office européen des brevets

(11) Publication number: 0 177 356 B1

# (12) EUROPEAN PATENT SPECIFICATION

(45) Date of publication of patent specification: 18.09.91 (51) Int. Cl.$^5$: A61K 31/195

(21) Application number: 85307088.6

(22) Date of filing: 03.10.85

## (54) Method for treatment of antidiuresis.

(30) Priority: 04.10.84 JP 208586/84

(43) Date of publication of application:
09.04.86 Bulletin 86/15

(45) Publication of the grant of the patent:
18.09.91 Bulletin 91/38

(84) Designated Contracting States:
AT BE CH DE FR GB IT LI NL SE

(56) References cited:

J. PHARM. PHARMACOL., vol. 33, no.12, December 1981, pages 778-782. H. ARAKI et al.: "Age-related changes in the chronotropic effect and the enzymicdecarboxylation of L-threo-3, 4-dihydroxyphenylserine in the rat heart."

EUR.J. CLIN. PHARMACOL., vol. 23, no. 5, 1982, pages 463-468, Springer Verlag. T. DUZUKI et al.: "Pharmacokinetic studies of oral-L-threo-3, 4-dihydroxyphenylserine in normal subjects and patients with familial amyloid plyneuropathy."

(73) Proprietor: SUMITOMO PHARMACEUTICALS COMPANY, LIMITED
40, Dosho-machi 2-chome
Higashi-ku Osaka-shi Osaka-fu(JP)

(72) Inventor: Katsube, Junki
10-20, Machikaneyamacho
Toyonaka-shi Osaka 560(JP)
Inventor: Nakamura, Mitsutaka
41, Mino-Aza-Yamagata
Kawanishi-shi Hyogo 666-01(JP)
Inventor: Maeda, Yukio
10-4-428, Sonehigashimachi 2-chome
Toyonaka-shi Osaka 561(JP)

(74) Representative: Kearney, Kevin David Nicholas et al
KILBURN & STRODE 30 John Street
London, WC1N 2DD(GB)

EP 0 177 356 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

J. PHARM. PHARMACOL., vol. 30, 1978, pages 456-458 H. ARAKI et al.: "Positive chronotropic effect of threo-3, 4-dihydroxyphenylserine as a procursor of noradrenaline in rat isolated atria."

J. PHARM. PHARMACOL., vol. 33, no. 12, December 1981, pages 772-777H. ARAKI et al.: "Pressor effect of L-threo-3, 4-dihydroxyphenylserine in rats."

JAPAN. J. PHARMACOL., vol. 28, October 1978, pages 747-753 I. OHMURA et al.: "Enzymatic decarboxylation of L-threo-3, 4-dihydroxyphenylserine in rat heart."

ARCH. INT. PHARMACODYN., vol. 222, no. 1, 1976, pages 94-102 R.G. PENDLETON et al.: "Studies concerning dopamine diuresis in the rats."

JOURNAL OF CLINICAL INVESTIGATION, vol. 52, February 1973, pages 502-511 R.W. SCHRIER et al.: "Mechanism of effect of alpha adrenergic stimulation with norepinephrine on renal water excretion."

JAPAN. J. PHARMACOL., vol. 39, Suppl. 1985, page 222, ref.no. 04G1348 M. NAKAMURA et al.: "A diuretic effect of L-threo-3, 4-dihydroxyphenylserine (L-threo-DOPS), a norepinephrine precursor in rats or mice."

J. PHARM. PHARMACOL., vol. 38, 1986, pages 533-534 J. KATSUBE et al.: "Diuretic effect of L-threo-3, 4-dihydroxyphenylserin, a noradrenaline precursor, in rats and mice."

## Description

The present invention relates to the use of threo-3-(3,4-dihydroxyphenyl)-serine (hereinafter referred to as threo-DOPS) for the manufacture of a medicament for the treatment of antidiuresis.

Diuretics directly act on the kidney and promote the excretion of sodium chloride and water from the kidney. There are various drugs having diuretic action and some of them have been clinically used. They are thiazide diuretics represented by chlorothiazide, loop diuretics represented by furosemide, and potassium-preserving diuretics represented by spironolactone and triamteren and so on.

The present invention provides a new type of treatment of antidiuresis different from those already known.

The 3-(3,4-dihydroxyphenyl)-serine related to the present invention is an aromatic amino acid abbreviated to DOPS. There are two configurational isomers, i.e. threo isomer (threo-DOPS) and erythro isomer (erythro-DOPS), and there are also optical isomers in each of them. That is, DOPS includes from stereoisomerism, L-threo-DOPS, D-threo-DOPS, L-erythro-DOPS and D-erythro-DOPS. In addition, in each of the threo-DOPS and erythro-DOPS, there is a racemic form (DL-isomer) which is an equivalent mixture of the D-isomer and the L-isomer.

It has been already known that L-DOPS undergoes decarboxylation by aromatic L-amino acid decarboxylase and is converted to noradrenaline (hereinafter referred to as NA) in vivo. Also it has been reported that, relating to the produced NA, a natural type 1-NA (originally present in a living body) is formed from L-threo-DOPS and an unnatrual type d-NA, from L-erythro-DOPS.

On the other hand, there have been some reports on the pharmacological actions of DOPS. That is, it has been reported that, from pharmacological tests using animals, erythro- or threo-DOPS has an antihypertensive or antidepressive effect (U.S.P. 3,920,728), L-threo-DOPS an inhibitory effect on harmaline induced tremor (Japanese Patent Publication (unexamined) No. 125630/1977), a pressor effect [Araki, H. et al, J. Pharm. Pharmac., 33 772 (1981)], or a positive chronotropic effect [Araki, H. et al., J. Pharm.Pharmac., 30, 456 (1978)] in rats, and L-erythro-DOPS a suppresive effect on psychomotor excitement (U.S.P. 4,529,603). On the other hand, based upon the results of clinical tests, there have been reported beneficial effects of DL- or L-threo-DOPS on orthostatic hypotension (U.S.P. 4,330,558) or the freezing phenomena of Parkinson's disease (U.S.P. 4,497,826).

Pressor effects have been reported for threo-3,4-dihydroxyphenylscrine in J. Pharm. Pharmacol., volume 33, No. 12, 1981 pages 778-782 and in Eur. J. Clin. Pharmacol, volume 23, No. 5, 1982, pages 463-468; influence on cardiac function in Japan. J. Pharmacol, volume 28, 1978, pages 747-753.

After having studied energetically for a long time various pharmacological actions which DOPS have, the present inventors have found that L- or DL- threo-DOPS has a significant diuretic action. There is not a report up to the present on such an action, and it is a finding first found out by the present inventors.

The mechanism of diuretic action of threo-DOPS is not yet clear. However, according to the studies of the present inventors, there are high possibilities that it is based upon ℓ-NA formed from L-threo-DOPS. That is, the present inventors observed that a concomitant use of a peripheral decarboxylase inhibitor (hereinafter referred to as DCI) abolished the diuretic action of L-threo-DOPS, and that the NA concentration in the kidney was significantly increased compared with those in other organs.

There have been several reports on the pharmacological action of ℓ-NA on the renal function up to the present; but it is very hard to foresee the diuretic action of L-threo-DOPS described in the present invention therefrom. For instance, it is said that the increase in the renal blood flow functions toward diuresis, however, it is generally known that ℓ-NA shows a contractive action on the renal blood vessel. In other words, ℓ-NA functions toward the decrease in the renal blood flow. There are contradictory reports on the action of ℓ-NA on the urinary volume, i.e. one is that it functions toward diuresis, and the other is toward antidiuresis. Such a confusion is presumed to be caused by the properties of ℓ-NA. That is, the action of ℓ-NA is violent and short-acting, and these properties make it difficult to grasp uniformly the effect of ℓ-NA on renal function.

On the contrary, the diuretic action of L-threo-DOPS comes out uniformly and prolongably when it is applied in an easy manner such as oral administration. This is one of the characteristic features of L-threo-DOPS.

The diuretic action of L-threo-DOPS of the present invention has as wide a clinical applicability as the existing diuretics. That is, it can be similarly applied to oedema which is the most general objective symptom of diuretics. Though there are anasarca and local dropsy, it can be considered from the characteristics of the present drug that the present drug is suitable for anasarca. Anasarca occurs in various forms, namely cardiac, hepatic, and renal anasarca. The present drug is most preferably applicable to cardiac anasarca, namely congestive heart failure, since the present drug appears to be converted to ℓ-NA

which has a cardiotonic action.

In addition, arginine vasopressin (hereinafter referred to as AVP) which is a hormone of the pituitary gland and is said to be an antidiuretic hormone (hereinafter referred to as ADH), as a factor to control the urinary volume,is well known. Also it is said that the acceleration of AVP action relates to one of the causes of cardiac or hepatic anasarca mentioned above. The present drugs are also thought likely to be effective against such antidiuretic conditions as myxedema, Addison's disease and other syndromes of inappropriate ADH secretion.

The fact that L- or DL-threo-DOPS of the present invention has very low toxicity supports the usefulness of the present drugs as practical medicines. In this connection, the acute toxicity by oral dosage to mouse, rat and dog is 10 g/kg or more.

L- or DL-threo-DOPS used in the present invention can be prepared by any of the known methods.

The threo-DOPS can be used in a form of a pharmaceutically acceptable acid addition salt. As acids useful to form such acid addition salts, there can be illustrated inorganic acids such as hydrochloric acid, hydrobromic acid and sulphuric acid, and organic acids such as fumaric acid, citric acid, tartaric acid and succinic acid.

The threo-DOPS, which is an active compound in the present invention, can be applied orally or parenterally in a dosage amount suitable to the individual need. That is, the amount of dosage for remedy can be orally applied in ordinary dosing forms such as tablets, capsules, syrups, and suspensions; or also materials in liquid forms such as solutions, emulsions, and suspensions thereof can be parenterally applied in a form of injections.

The drugs of suitable dosage types mentioned above can be prepared by combining an active compound with ordinary allowable pharmaceutically acceptable carriers, vehicles, binders and stabilizers. When used in the form of injections, allowable buffers, dissolution aids and isotonic agents, may be added.

The amount of dosage and the frequency of dosage of threo-DOPS used in the present application are different depending upon the form of dosage and the extent of the symptoms requiring remedy; however, for instance, in the case of oral administration, it can be applied in an amount of 0.1-4 g per adult per day at once or several times dividedly.

In the case of intravenous injection, it can be applied in an amount of 0.1-22 g per adult per day at once or several times dividedly.

The invention may be put into practice in various ways and a number of specific embodiments will be described to illustrate the invention with reference to the accompanying examples, and the accompanying drawings in which:

Figure 1 is a diagram which shows the effect of L-threo-DOPS on urinary volume of rats. The ordinate represents the urinary volume (ml/kg/3 hr) and the abscissa, the amount of dosage of L-threo-DOPS (mg/kg); and

Figure 2 is a diagram which shows the effect of L-threo-DOPS on urinary volume of mice. The ordinate represents the urinary volume (ml/kg/3 hr) and the abscissa, the amount of dosage of L-threo-DOPS (mg/kg).

In Figures 1 and 2, the mark * represents $p < 0.05$ (compared with the control group).

EXPERIMENTAL EXAMPLE 1

Effect of L-threo-DOPS on urinary volume and electrolytes in urine of rats

Three rats starved for 17 hours were arranged to form a group. Ten mg/kg, 30 mg/kg, and 100 mg/kg of L-threo-DOPS were suspended in 0.5% aqueous solution of methyl cellulose, respectively, and orally administered together with 25 ml/kg of isotonic sodium chloride solution. A control group was dosed with only a 0.5% aqueous solution of methyl cellulose and isotonic sodium chloride solution. The urinary volume was measured for 3 hours after the administration of the dose. In addition, among electrolytes in the urine, amounts of sodium ion ($Na^+$) and potassium ion ($K^+$) were measured by a flame photometer, and the amount of chlorine ion ($Cl^-$) by the method of Zall et al. (Anal. Chem. 28, 1665).

Increase in the urinary volume was observed in accordance with the amount of dosage of L-threo-DOPS, as shown in Figure 1.

The effect of the dose on electrolytes in the urine is as shown in Table 1. In Table 1, values are represented in units of mEq/kg/3 hr and as the average of 5 groups ± S.E.

Table 1 shows that the amount of $Na^+$ in urine significantly increases in the groups wherein the dose is 10 mg/kg or more of L-threo-DOPS; the amount of $K^+$ is not so much influenced by L-threo-DOPS but a significant increase is recognized only in the group wherein the dose was 100 mg/kg. The amount of $Cl^-$ in

the urine is significantly increased only in the groups wherein the dose is 30 mg/kg or more of L-threo-DOPS.

TABLE 1

Action of L-threo-DOPS on electrolytes in urine

| Amount of dosage (mg/kg) | $Na^+$ | $K^+$ | $Cl^-$ |
|---|---|---|---|
| 0 (control | 0.91 ± 0.08 | 0.30 ± 0.04 | 1.54 ± 0.05 |
| 10 | 1.58 ± 0.12* | 0.32 ± 0.03 | 1.89 ± 0.14 |
| 30 | 2.45 ± 0.32* | 0.37 ± 0.05 | 2.97 ± 0.25* |
| 100 | 3.15 ± 0.07* | 0.45 ± 0.03* | 3.24 ± 0.07* |

* $P < 0.05$ (compared with the control group)

EXPERIMENTAL EXAMPLE 2

Effect of L-threo-DOPS on urinary volume of mice

Ten mice starved for 17 hours were arranged to form a group. Ten mg/kg, 30 mg/kg, and 100 mg/kg of L-threo-DOPS were suspended in a 0.5% aqueous solution of methyl cellulose, respectively, and orally administered together with 25 mg/kg of isotonic sodium chloride solution. A control group was dosed with only a 0.5% aqueous solution of methyl cellulose and isotonic sodium chloride solution.

The results are as shown in Figure 2. L-threo-DOPS increases the urinary volume in accordance with the amount of the dose, and the increase in urinary volume is significant in the group wherein the dose is 100 mg/kg.

EXPERIMENTAL EXAMPLE 3

Effect on diuretic action of L-threo-DOPS in combination with DCI.

Investigation was made on the effect of L-threo-DOPS on diuretic action when DCI, benserazide or carbidopa was given by oral administration at the same time, in the same manner as Experimental Example 1.

As a result, as shown in Table 2 (values of urinary volume are represented in units of ml/kg/3 hr and as the average of 5 groups ± S.E.), the diuretic action of L-threo-DOPS is significantly decreased by using benserazide or carbidopa together therewith, and the possibility is suggested that the diuretic action of L-threo-DOPS is revealed via NA.

TABLE 2

Effect of periphery decarboxylase inhibitor on diuretic action of L-threo-DOPS

| Procedure | Urinary volume |
|---|---|
| (Control) | 15.9 ± 1.8 |
| L-threo-DOPS (30 mg/kg) | 51.9 ± 3.0* |
| Benserazide (1 mg/kg) + L-threo-DOPS (30 mg/kg) | 20.4 ± 0.8** |
| Carbidopa (1 mg/kg) + L-threo-DOPS (30 mg/kg) | 13.7 ± 2.1** |

* $P < 0.05$ (compared with the control group)

** $P < 0.05$ (compared with the group to which only L-threo-DOPS was dosed)

EXPERIMENTAL EXAMPLE 4

Effect of L-threo-DOPS on the amount of NA in the kidney and heart of rats.

Rats were dosed with 30 mg/kg of L-threo-DOPS by oral administration, and the amount of NA in the kidney and heart after 1 hour was measured.

The method for the measurement was generally in accordance with the method of Suzuki, et al. (Europ. J. Clin. Pharmacol., 23, 463, 1982).

The results are shown in Table 3 wherein values are represented as the amount of NA in ng/g wet weight and as the average of 5 rats ± S.E. Oral administration of L-threo-DOPS (30 mg/kg) markedly increases the amount of NA in the kidney but makes no influence on the amount of NA in the heart.

TABLE 3

Amounts of NA in the kidney and heart after dosage with L-threo-DOPS.

| | Kidney | Heart |
|---|---|---|
| Control group | 0.17 ± 0.01 | 0.98 ± 0.04 |
| Group of which L-threo-DOPS was dosed | 1.80 ± 0.35* | 1.00 ± 0.06 |

* $P < 0.05$ (compared with the control group)

The preparation of DL-threo-DOPS is described in GB 2000779. Thus DOPS may be prepared by condensing 3,4-dibenzyloxybenzaldehyde and glycine and then subjecting the resulting 3-(3,4-dibenzyloxyphenyl)serine to debenzylation. The DOPS is obtained as a mixture of diastereoisomers (i.e. the threo isomer and the erythro isomer).

A single isomer can be isolated from a mixture of the threo and erythro isomers of 3-(3,4 dibenzyloxyphenyl)-serine, by treating the mixture with a mineral acid, and separating the mineral salt of a single isomer using a technique which depends on the difference in solubility of the mineral acid salts of the two isomers. The threo-3-(3,4-dibenzyloxyphenyl)serine or a mineral acid salt thereof obtained by the above method is subjected to debenzylation to give threo-DOPS.

In practical terms, the procedure is as follows.

When the mixture of the mineral acid salts of the threo- and erythro isomers is subjected to fractional crystallization after isolating the mixture from the reaction mixture, threo- and erythro-3-(3,4-dibenzyloxyphenyl)serine is reacted with one mole or more, preferably one to two moles, of a mineral acid per mole of the threo and erythro isomers, in an appropriate solvent, such as water, methanol, ethanol, acetone, N,N-dimethylformamide, or a mixture thereof. This reaction is generally carried out at room temperature or an elevated temperature, preferably from 20 to 60 °C. The mixture of the resulting mineral acid salts of the threo and erythro isomers can be isolated from the reaction mixture by evaporating the solvent under reduced pressure or by adding a solvent, in which the mineral acid salts are sparingly soluble, such as diethyl ether, toluene or n-hexane. In this method, the starting threo- and erythro-3-(3,4-dibenzyloxyphenyl)-serine may have any molar ratio of the threo isomer and erythro isomer. Suitable mineral acids include hydrochloric acid, hydrobromic acid, hydroiodic acid and nitric acid. The hydrochloric acid is preferred because of its price and ease of handling.

The mixture of the mineral acid salts thus isolated is then subjected to fractional crystallization by a conventional method to give the relatively insoluble mineral acid salt of threo-3-(3,4-dibenzyloxyphenyl)-serine. The fractional crystallization can generally be carried out by dissolving the mixture in a solvent which is heated to a temperature a little higher than that sufficient to dissolve it, and subsequently gradually cooling the solution to room temperature or lower, preferably 30° to ±20 °C, to crystallize out the mineral acid salt of the threo isomer, while the mineral acid salt of the erythro isomer remains in solution. The particular solvent to be used for the fractional crystallization is not important, unless the particular mineral acid salt is decomposed or hydrolyzed. However, it is preferable not to use a solvent which cannot or can only slightly dissolve the mineral acid salts, such as diethyl ether or toluene, or a solvent containing a large amount of diethyl ether or toluene. Examples of suitable solvents for the fractional crystallization are water, ethanol, isopropanol, acetone, ethyl acetate, and mixtures thereof.

The thus separated mineral acid salt of threo-3-(3,4-dibenzyloxyphenyl)serine may be converted into free threo-3-(3,4-dibenzyloxyphenyl)serine by a conventional process, for instance, by treating the mineral acid salt with a base, such as sodium hydroxide, sodium acetate or diethylamine.

## EXAMPLE 5

A mixture of threo- and erythro-3-(3,4-dibenzyloxyphenyl)serine (molar ratio of threo isomer: erythro isomer = about 3:1,5 g) is dissolved in a mixture of methanol (30 ml) and 3N hydrochloric acid (10 ml), and the mixture is distilled under reduced pressure to remove the solvent, and thereby threo-and erythro-3-(3,4-dibenzyloxyphenyl)serine hydrochloride are obtained. The salts thus obtained are recrystallized from ethanol to give threo-3-(3,4-dibenzyloxyphenyl)serine hydrochloride (2.7 g), m.p. 150° to 154 °C. This is then de-benzylated in conventional manner to give DL-threo-DOPS.

The preparation of L-threo-DOPS is as described in USP 4319040. Thus optically active L-threo-DOPS is made by firstly subjecting a racemic protected DOPS to optical resolution with a specific resolving reagent and then subjecting the resulting optically active protected DOPS to hydrogenolysis.

The process comprises subjecting racemic threo-3-(3,4-dibenzyloxyphenyl)-N-carbobenzyloxyserine of the formula:

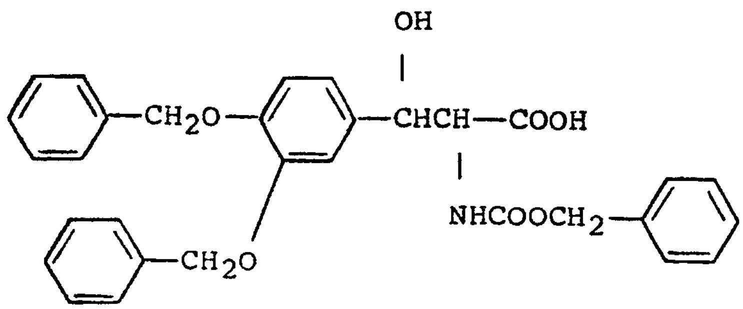

[I]

to optical resolution with an optically active amino alcohol derivative of the formula:

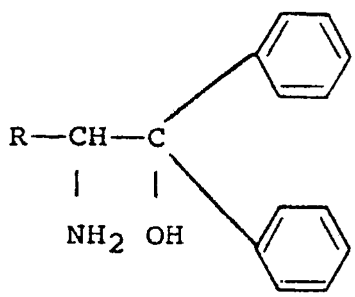

[II]

wherein R is methyl, isopropyl or isobutyl, and then subjecting the resulting optically active L-threo-3-(3,4-dibenzyloxyphenyl)-N-carbobenzyloxyserine to hydrogenolysis to give the desired optically active L-threo-DOPS.

The starting racemic threo-3-(3,4-dibenzyloxyphenyl)-N-carbobenzyloxyserine[I] is a known compound and can easily be obtained by introducing a carbobenzyloxy group onto the amino group of racemic threo-3-(3,4-dibenzyloxyphenyl)serine. The optically active amino alcohol derivatives [II] used as the resolving reagent are also known [cf. J. Chem. Soc., 287 (1925), 785 (1926); J. Pharm. Soc., Japan 48, 46 (1928)] and can easily be produced from an optically active α-amino acid by a single step as shown in the following reaction scheme A:

Reaction Scheme A

[II]

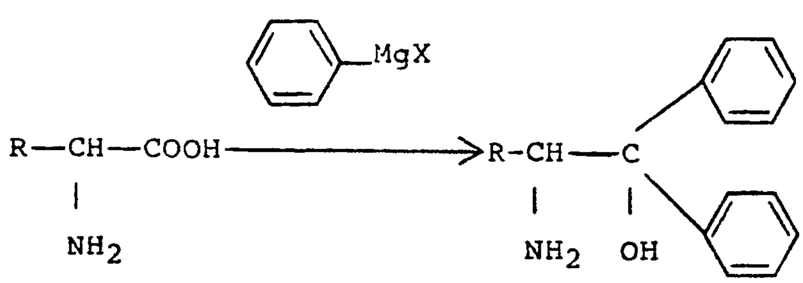

wherein R is as defined above, and X is a halogen atom.

EXAMPLE 6

Racemic threo-3-(3,4-dibenzyloxyphenyl)-N-carbobenzyloxyserine (65 g) is dissolved in methanol (650 g) at 50° C, and therein is dissolved S-2-amino-1,1-diphenylpropanol (28.0 g). The mixture is cooled to 30° C over a period of one hour. The mixture is again heated to 50° C and is then cooled to 25° C over a period of 2 hours and is agitated under ice-cooling for 3 hours. The precipitated crystals are collected to obtain L-threo-3-(3,4-dibenzyloxyphenyl)-N-carbobenzyloxyserine S-2-amino-1,1-diphenylpropanol salt (40.5 g). Melting point 151°-153° C, $[\alpha]_D^{20}$—30.5° (c = 1, tetrahydrofuran).

The salt (20 g) thus obtained is decomposed by treating with 3% hydrochloric acid to give L-threo-3-(3,4-dibenzyloxyphenyl)-N-carbobenzyloxyserine in the crystalline form (13.5 g). Melting point 130°-132° C, $[\alpha]_D^{20}$ - 18.3° (c = 1, methanol).

EXAMPLE 7

L-Threo-3-(3,4-dibenzyloxyphenyl)-N-carbobenzyloxyserine,$[\alpha]_D^{20}$ - 19.3° (c = 1, methanol) (20 g) is dissolved in a mixture of methanol (300 g) and water (10 g). To the solution are added concentrated hydrochloric acid (4.9 g) and 5% palladium-active carbon (0.6 g), and the mixture is subjected to hydrogenolysis with hydrogen gas at room temperature under atmospheric pressure. After absorption of hydrogen gas is no longer observed, the reaction mixture is filtered to remove undissolved materials, and the mixture is neutralized with a 5 N aqueous sodium hydroxide solution. The mixture is agitated under ice-cooling for 2 hours. The precipitated crystals are collected by filtration to give L-threo-3-(3,4-dihydroxyphenyl)serine (8.0 g). Melting point 203°-206° C (decomp.).

The compound thus obtained is recrystallized from water (450 g) containing L-ascorbic acid (80 mg) to give a purified product (5.7 g). Melting point 229°-232° C (decomp.), $[\alpha]_D^{20}$-42.0° (c = 1, 1 N hydrochloric acid).

**Claims**

1. The use of L- or DL-threo-3-(3,4-dihydroxyphenyl)-serine or a pharmaceutically acceptable salt thereof for the manufacture of a medicament for the treatment of antidiuresis.

2. The use as claimed in Claim 1 in which the salt is an acid addition salt.

3. The use as claimed in Claim 1 in which the serine is L-threo-3-(3,4-dihydroxyphenyl)-serine.

**Revendications**

1. L'utilisation de la L-thréo-3-(3,4-dihydroxyphényl)-sérine) ou de la DL-thréo-3-(3,4-dihydroxyphényl)-sérine) ou de l'un de ses sels acceptables pharmaceutiquement, pour la fabrication d'un médicament destiné au traitement de l'antidiurèse.

2. Utilisation suivant la revendication 1, dans laquelle le sel est un sel d'addition avec un acide.

3. Utilisation suivant la revendication 1, dans laquelle la sérine est la L-thréo-3-(3,4-dihydroxyphényl)-sérine).

**Patentansprüche**

1. Verwendung von L- oder DL-threo-3-(3,4-dihydroxyphenyl)-serin oder eines pharmazeutisch verträglichen Salzes davon für die Herstellung eines Medikaments zur Behandlung von Antidiuresis.

2. Verwendung nach Anspruch 1, wobei das Salz ein Säureadditionssalz ist.

3. Verwendung nach Anspruch 1, wobei das Serin L-threo-3-(3,4-dihydroxyphenyl)-serin ist.

FIG. 1

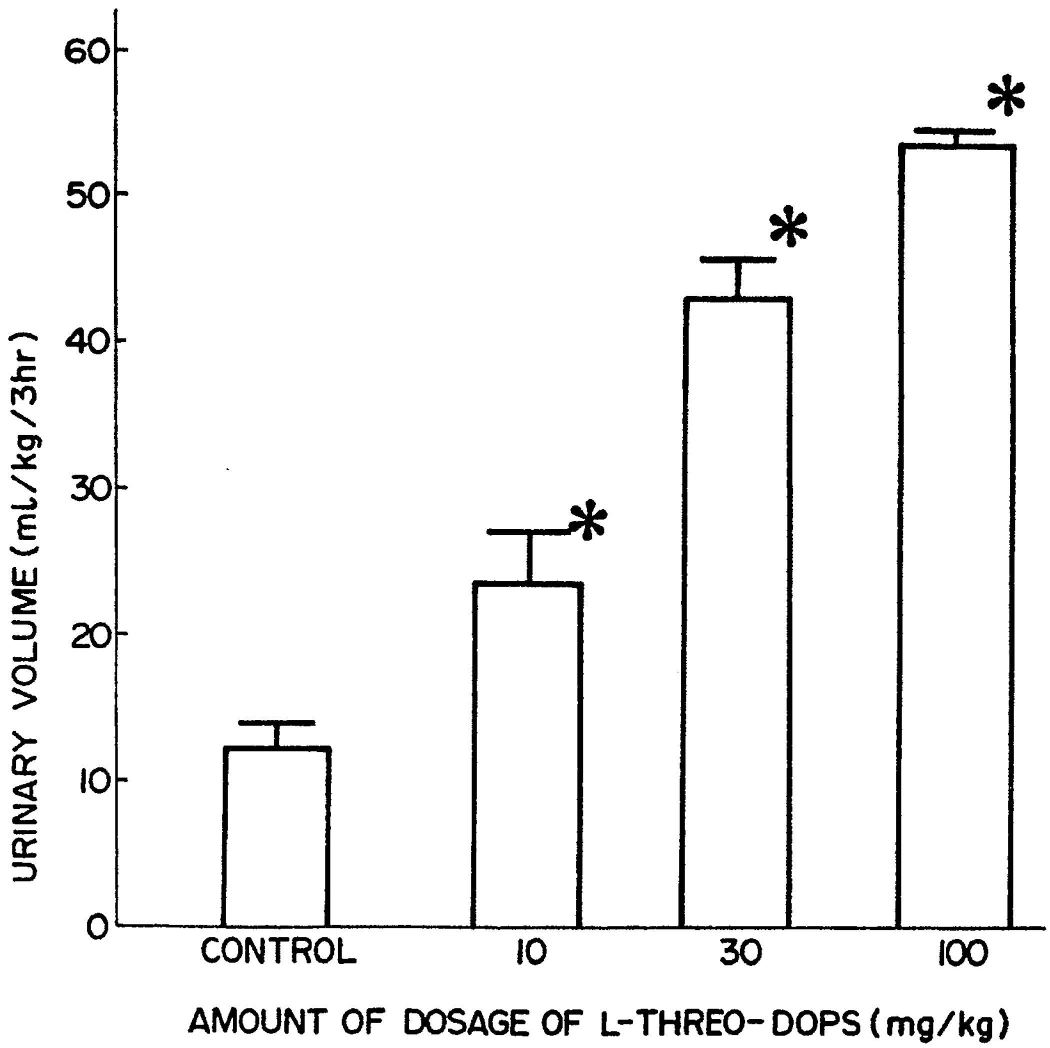

URINARY VOLUME (mL/kg/3hr)
0
10
20
30
40
50
60
*
*
*
CONTROL
10
30
100
AMOUNT OF DOSAGE OF L-THREO-DOPS (mg/kg)

FIG. 2

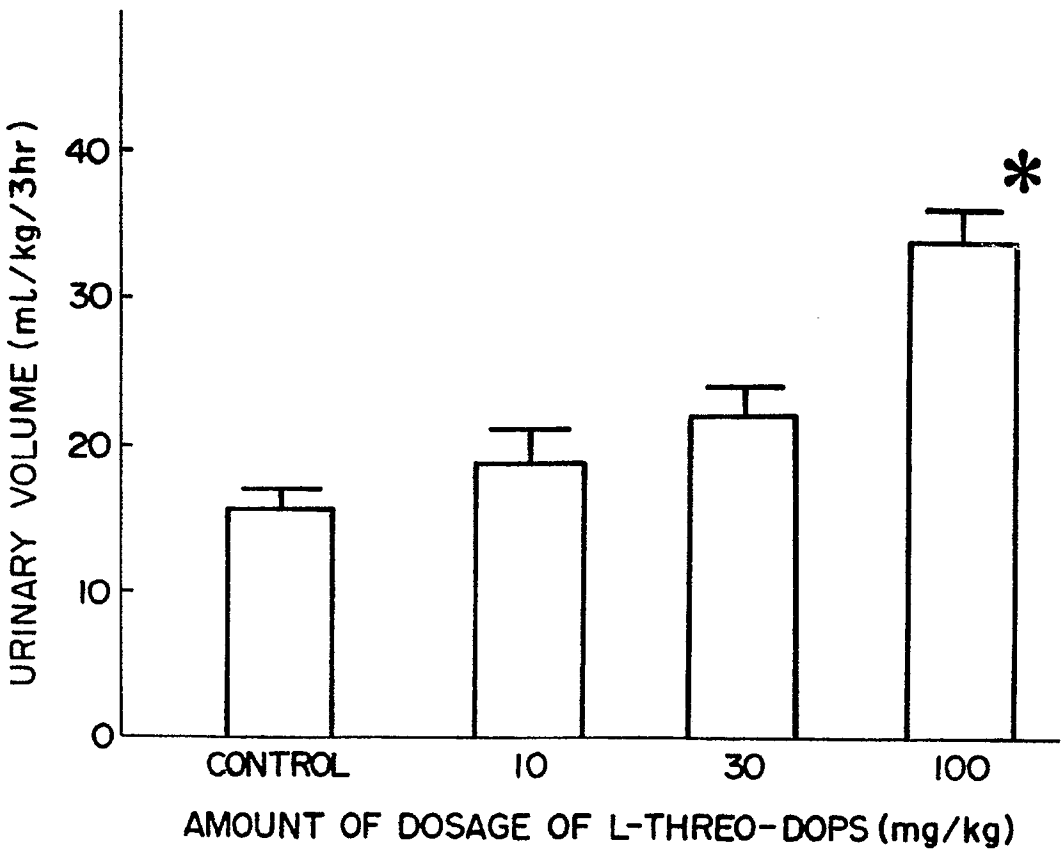
URINARY VOLUME (mL/kg/3hr)
40
30
20
10
0
*
CONTROL
10
30
100
AMOUNT OF DOSAGE OF L-THREO-DOPS (mg/kg)